# EUROPÄISCHE PATENTSCHRIFT

(11) **EP 3 964 160 B1**
(45) Veröffentlichungstag und Bekanntmachung des Hinweises auf die Patenterteilung: **04.09.2024**
(21) Anmeldenummer: 21201923.6
(22) Anmeldetag: 11.07.2019
(51) Int. Cl.: A61B 50/30, B65D 45/24, A61B 50/00

(54) **CONTAINERVERSCHLUSS**
CONTAINER CLOSURE
FERMETURE DE RÉCIPIENT

(30) Priorität: 13.07.2018 DE 102018117046
(43) Veröffentlichungstag der Anmeldung: 09.03.2022
(62) Teilanmeldung aus: 19748723.4
(73) Patentinhaber: Aesculap AG, 78532 Tuttlingen (DE)
(72) Erfinder: THOMAS, Stefan, 78532 Tuttlingen (DE); ELISCH, Andreas, 78655 Dunningen (DE); EISKANT, Karl-Heinz, 78532 Tuttlingen (DE)
(74) Vertreter: Winter, Brandl - Partnerschaft mbB

(56) Entgegenhaltungen:
- WO-A2-2008/078169
- DE-U1- 202012 102 803
- FR-A- 1 578 189
- US-A- 5 706 968
- US-A1- 2006 042 897

## Beschreibung

Die vorliegende Erfindung betrifft einen Verschluss bzw. Verschlussmechanismus /Verriegelungsmechanismus für einen / eines medizinischen Sterilcontainer(s) insbesondere zum Verriegeln eines Deckels auf einer Containerwanne.

### Hintergrund der Erfindung

Sterilcontainer der vorliegenden Gattung werden u.a. zur Sterilisation medizinischer Instrumente eingesetzt. Hierfür hat der Sterilcontainer eine Aufnahme- oder Containerwanne, in die zu sterilisierende Instrumente eingelegt werden können, einen Containerdeckel zum fluiddichten Verschließen des Containers sowie einen Verschluss/Verschlussmechanismus (Verriegelungsmechanismus), mittels welchem der Deckel mit der Containerwanne fest (fluiddicht) verriegelt werden kann. Des Weiteren ist der Sterilcontainer in der Regel mit einem Ventilmechanismus ausgebildet/ausgerüstet, der das Ansteigen eines Container-Innendrucks über einen den Container ggf. beschädigenden vorbestimmten/vorbestimmbaren Wert während eines Sterilisationsprozesses in einem Autoklav verhindert.

### Stand der Technik

Aus dem Stand der Technik beispielsweise gemäß einschlägiger Produkte der vorliegenden Anmelderin selbst sind (medizinische) Sterilcontainer der vorstehend beschriebenen Gattung bekannt, die eine mittels eines Containerdeckels verschließbare Containerwanne sowie einen Verschluss-/Verriegelungsmechanismus haben, über welchen der Deckel mit der Wanne fluiddicht verriegelbar ist. Im Containerdeckel ist ferner eine Ventileinrichtung vorgesehen, die ein Einströmen von Umgebungsgas in den Containerinnenraum über eine (entkeimende) Filtereinrichtung und ein Ausströmung von Container-Innenraumgas in die Umgebung unter Umgehung der Filtereinrichtung erlaubt, um den Container-Innendruck gegenüber dem Umgebungsdruck unterhalb eines vorbestimmten/vorbestimmbaren Werts zu halten. Entscheidend für die korrekte Funktion dieser Ventileinrichtung ist die sichere und fluiddichte Verriegelung des Deckels auf/an der Containerwanne, um ungewollte Leckagen in/aus dem Container an der Kontaktstelle zwischen Wanne und Deckel zu vermeiden.

Als eine besonders vorteilhafte Ausgestaltung eines solchen Verschluss-/Verriegelungsmechanismus hat sich der sogenannte Bügelverschluss erwiesen. Dieser hat einen schwenkbar beispielsweise am Deckel gelagerten Betätigungshebel, an dem ein Bügel oder eine Kralle/Spannpratze angelenkt ist, der/die mit einer seitlich, beispielsweise an der Containerwanne vorragenden Leiste in hinterschneidenden Eingriff kommt und so bei einem Umklappen des Betätigungshebels den Containerdeckel gegen die Containerwanne zieht. Zwischen Deckel und Wanne ist ein Dichtungsring angeordnet, der bei Anziehen des Deckels gegen die Wanne und den Deckel gequetscht wird und so den Kontaktbereich zwischen Wanne und Deckel abdichtet. Dabei können auch geringe Toleranzen ausgeglichen werden.

Wichtig hierbei ist, dass
- an der Stirnseite des Behälterunterteils (Containerwanne) trotz des Verschlussmechanismus/Verschlusseinheit eine möglichst große Fläche beispielsweise zur Kennzeichnung des Behälters/Containers frei bleibt,
- ein versehendliches Öffnen der Verschlusseinheit weit möglichst verhindert wird,
- auch größere Fertigungstoleranzen ausgeglichen werden können und
- eine Mindestanzugskraft zwischen Deckel und Wanne gewährleistet ist.

Darüber hinaus wäre es wünschenswert, dass
- die Zugkraft nicht plötzlich sondern fortlaufend mit fortschreitendem Umlegen des Betätigungshebels zunimmt, insbesondere um Beschädigungen des Verschlussmechanismus und/oder der Dichtung zu vermeiden,
- der Verschlussmechanismus gleichzeitig auch eine Überdruckventilfunktion übernehmen kann,
- der Betätigungshebel an einer gut zugänglichen Stelle, vorzugsweise an der Oberseite des Containerdeckels liegt, um ein seitliches Vorragen auch während des Öffnungs-/Schließvorgangs zu vermeiden und die Stirnseite der Containerwanne nicht abzudecken und
- der Verschlussmechanismus in seiner geöffneten / entriegelten Endposition möglichst stabil fixiert ist.

FR 1 578 189 A offenbart einen Verschluss-/Verriegelungsmechanismus gemäß dem Oberbegriff des Anspruchs 1.

US 2006/042897 A1 offenbart einen Verschluss-/ Verriegelungsmechanismus.

### Kurzbeschreibung der Erfindung

Angesichts vorstehender Beschreibung des Stands der Technik ist es die Aufgabe der vorliegenden Erfindung, einen funktionalen Verschluss-/Verriegelungsmechanismus (Verschlusseinheit) für einen / eines Sterilcontainer(s) vorzugsweise der medizinischen Bauart bereitzustellen, mittels welchem die vorstehend genannten wünschenswerten Eigenschaften möglichst sämtlich erreichbar sind.

Diese Aufgabe wird durch einen Verschluss-/Verriegelungsmechanismus (Verschlusseinheit), vorzugsweise für einen / eines medizinischen Sterilcontainer(s) mit den Merkmalen des Patentanspruchs 1 bzw. durch einen (medizinischen) Sterilcontainer mit einem solchen Verschluss-/Verriegelungsmechanismus (Verschlusseinheit) gelöst. Vorteilhafte Ausgestaltungen/Weiterbildungen der Erfindung sind Gegenstand der Unteransprüche.

Die vorliegende Erfindung betrifft demzufolge einen Verschluss-/Verriegelungsmechanismus mit einem vorzugsweise schwenkbar gelagerten Betätigungshebel, einem Zugbügel oder Kralle/Spannpratze, die mit dem Betätigungshebel direkt oder indirekt gekoppelt ist und einem eine Hinterschneidung ausbildenden Eingriffselement (Leiste), mit welchem der Zugbügel oder Kralle in Wirkeingriff bringbar ist, um bei einer Betätigung des Betätigungshebels eine Zugkraft auf das Eingriffselement auszuüben. Erfindungsgemäß ist der Zugbügel oder Kralle quer, vorzugsweise in einem rechten Winkel, zur auszuübenden Zugkraft (Kraftvektor) verschiebbar gelagert, wobei eine Feder relativ zu dem Zugbügel oder Kralle derart in einer Wirkposition angeordnet ist, dass diese erst bei einer vorbestimmten Betätigungsstellung des Betätigungshebels und demzufolge einer vorbestimmten/vorbestimmbaren Verschiebeposition des Zugbügels oder Kralle, in welcher diese(r) das Eingriffselement bereits hintergreift, eine Druckkraft auf den Zugbügel oder Kralle in Richtung (im Wesentlichen parallel zu) der auszuübenden Zugkraft anlegt.

In anderen Worten betrifft die vorliegende Erfindung einen Verschluss-/Verriegelungsmechanismus für einen Sterilcontainer bestehend aus einer Containerwanne und einem mittels des Verschluss-/Verriegelungsmechanismus mit der Containerwanne vorzugsweise fluiddicht verriegelbaren, auf einer Oberkante der Containerwanne angeordneten Containerdeckel, mit: einem Betätigungshebel, der an einem aus dem Containerdeckel und der Containerwanne vorzugsweise schwenkbar gelagert ist, einem Zugbügel oder einer Kralle/Spannpratze, der oder die mit dem Betätigungshebel direkt oder indirekt gekoppelt ist; und einem eine Hinterschneidung ausbildenden Eingriffselement seitlich an dem anderen aus der Containerwanne dem Containerdeckel, mit welchem der Zugbügel oder Kralle/Spannpratze in hinterschneidenden Wirkeingriff bringbar ist, um bei einer Betätigung des Betätigungshebels in eine Verriegelungsposition eine Zugkraft auf das Eingriffselement für ein Zusammenpressen der Containerwanne und des Containerdeckels auszuüben. Der Zugbügel oder Kralle/Spannpratze ist quer, vorzugsweise in einem im Wesentlichen rechten Winkel, zur auszuübenden Zugkraft verschiebbar an dem Containerdeckel oder der Containerwanne gelagert. Eine Feder oder dergleichen Vorspannelement ist vorgesehen, die relativ zu dem Zugbügel oder Kralle/Spannpratze derart in einer Wirkposition vorzugsweise Abstandsposition in Zugbügel-Verschieberichtung angeordnet ist, dass diese erst bei einer vorbestimmten Betätigungsstellung des Betätigungshebels und demzufolge einer vorbestimmten/vorbestimmbaren Verschiebeposition des Zugbügels oder Kralle/Spannpratze, in welcher der Zugbügel oder Kralle/Spannpratze das Eingriffselement bereits hintergreift, eine Druckkraft auf den Zugbügel oder Kralle/Spannpratze in Richtung der auszuübenden Zugkraft anlegt.

Demnach bewirkt die Betätigung des Betätigungshebels nur indirekt ein Anziehen des Containerdeckels gegen die Containerwanne, indem der beispielsweise deckelseitige Zugbügel/Kralle mittels des Betätigungshebels in einer ersten Bewegungsrichtung in Wirkposition bezüglich des beispielsweise wannenseitigen Eingriffselements gebracht/verschoben wird, in der dann eine Feder eine Druckkraft auf den Zugbügel/Kralle in einer Richtung vorzugsweise im Wesentlichen quer zu dessen Bewegungsrichtung anlegt, die demzufolge zumindest anteilsmäßig parallel zur beabsichtigten Zugkraftrichtung ausgerichtet ist. Dadurch wird grundsätzlich eine federkraftabhängige und damit vordefinierte Spannkraft zwischen Deckel und Wanne gewährleistet, die nicht von der Betätigungskraft auf den Betätigungshebel abhängig ist. Die Handhabung des erfindungsgemäßen Verschlussmechanismus ist daher gegenüber dem Stand der Technik leichter und dessen Verschlusssicherheit höher. Darüber hinaus ist es möglich, dass sich der Deckel bei Erreichen eines bestimmten Überdrucks innerhalb des Containers gegenüber der Atmosphäre entgegen der Federkraft öffnet und somit Container-Innendruck in die Atmosphäre entweichen kann.

Konstruktiv umsetzbar ist das vorstehende Erfindungsprinzip bevorzugt dadurch, dass der Betätigungshebel über zumindest einen daran angelenkten Stößel (Pleuel) mit dem Zugbügel oder Kralle/Spannpratze für eine Transformation seiner Schwenkbewegung in eine Translationsbewegung des Zugbügels oder Kralle/Spannpratze gekoppelt ist. D.h. der Betätigungshebel ist nach Art einer Kniehebelkonstruktion mit dem Zugbügel gekoppelt.

Vorteilhaft ist es, dass der Betätigungshebel über zumindest einen, vorzugsweise zwei axial beabstandete Scharnierbolzen am Containerdeckel (an dessen Oberseite) angelenkt ist, derart, dass sich der Zugbügel oder Kralle/Spannpratze bezüglich des Betätigungshebels unterhalb des zumindest einen, vorzugsweise zwischen den zwei axial beabstandeten Scharnierbolzen erstreckt. D.h. der Zugbügel befindet sich zwischen Containerdeckel (Oberseite) und Betätigungshebel.

Weiter vorteilhaft ist es, dass der Betätigungshebel an seinem einen freien Endabschnitt eine Betätigungsplatte/Taste oder Betätigungsbügel/Griff und an seinem anderen freien Endabschnitt eine Niederdrücklasche bildet, welche in aufgeklapptem Zustand des Betätigungshebels den Zugbügel oder Kralle/Spannpratze bezüglich der/des Scharnierbolzen(s) in Richtung hin zum Containerdeckel (Oberseite) niederdrückt und in eingeklapptem Zustand des Betätigungshebels den Zugbügel oder Kralle/Spannpratze für dessen Bewegung hin zu dem/der Scharnierbolzen (weg vom Containerdeckel) freigibt.

Da der Zugbügel oder Kralle an seinem einen Endabschnitt einen, den Containerdeckel randseitig umgreifenden Haken bildet, welcher mit dem wannenseitigen Eingriffselement (Leiste) verrasten soll, untergreift der Haken in der niedergedrückten Stellung des Zugbügels/Kralle im Wesentlichen berührungsfrei das Eingriffselement (Leiste) und legt sich an diesem erst mit Erreichen der vorbestimmten Verschiebeposition an.

Zur Erzielung einer Federkraft ist es hierbei vorteilhaft, dass um den/die Scharnierbolzen bzw. deren Längsachse herum die Feder, bevorzugt als Blattfeder, sichel- oder teilkreisförmig gebogen ist, derart, dass sich diese an ihrer einen freien Blattfederkante (Federendabschnitt) an einer bezüglich des/der Scharnierbolzen(s) unteren Seite des Zugbügels oder Kralle/Spannpratze anliegt und an ihrer anderen freien Blattfederkante (Federendabschnitt) gegen eine in Schieberichtung des Zugbügels oder Kralle/Spannpratze sich erstreckende Zunge/Vorsprung/Abschnitt des Zugbügels oder Kralle/Spannpratze erst dann andrückt, wenn dieser die vorbestimmte Verschiebeposition erreicht, um so an ihrer einen freien Blattfederkante (Federendabschnitt) die Druckkraft auf den Zugbügel oder Kralle/Spannpratze zu erzeugen.

Alternativ hierzu kann es vorgesehen sein, dass die Feder vorzugsweise in Form einer Bügelfeder am Zugbügel oder Kralle/Spannpratze fixiert ist, derart dass sich diese(r) bei einer Verschiebung des Zugbügels oder Kralle/Spannpratze am Containerdeckel federelastisch abstützt, wenn der Zugbügel oder Kralle/Spannpratze die vorbestimmte Verschiebeposition erreicht, um so die Druckkraft auf den Zugbügel oder Kralle/Spannpratze zu erzeugen.

Weiter alternativ kann es vorgesehen sein, dass die Feder, vorzugsweise in Form einer gewellten oder bogenförmigen Blattfeder, am Containerdeckel fixiert ist, derart, dass diese vom Zugbügel oder Kralle/Spannpratze überglitten wird, zumindest wenn dieser die vorbestimmte Verschiebeposition erreicht, um so die Druckkraft auf den Zugbügel oder Kralle/Spannpratze anzulegen.

### Kurzbeschreibung der Figuren

Die Erfindung wird nachstehend anhand bevorzugter Ausführungsbeispiele unter Bezugnahme auf die begleitenden Figuren näher erläutert.
Fig. 1 zeigt einen Querschnitt eines Verschluss-/Verriegelungsmechanismus gemäß einem ersten bevorzugten Ausführungsbeispiel der Erfindung in vollständig entriegeltem (unverschlossenen) Zustand,
Fig. 2 zeigt den Verschluss-/Verriegelungsmechanismus gemäß erstem Ausführungsbeispiel zu Beginn eines Verriegelungsvorgangs,
Fig. 3 zeigt den Verschluss-/Verriegelungsmechanismus gemäß erstem Ausführungsbeispiel im weiteren Verlauf des Verriegelungsvorgangs,
Fig. 4 zeigt den Verschluss-/Verriegelungsmechanismus gemäß erstem Ausführungsbeispiel am Ende des Verriegelungsvorgangs,
Fig. 5 zeigt eine Perspektivenansicht des Verschluss-/Verriegelungsmechanismus gemäß erstem Ausführungsbeispiel, wie er an der Oberseite eines Containerdeckels montiert ist,
Fig. 6 zeigt eine Seitenansicht eines Verschluss-/Verriegelungsmechanismus gemäß einem zweiten Ausführungsbeispiel der vorliegenden Erfindung.
Fig. 7 zeigt eine Perspektivenansicht des Verschluss-/Verriegelungsmechanismus gemäß zweitem Ausführungsbeispiel in entriegeltem (unverschlossenem) Zustand,
Fig. 8 zeigt eine Perspektivenansicht des Verschluss-/Verriegelungsmechanismus gemäß zweitem Ausführungsbeispiel in verriegeltem (verschlossenem) Zustand,
Fig. 9 zeigt eine Perspektivenansicht eines Verschluss-/Verriegelungsmechanismus gemäß einem dritten Ausführungsbeispiel der vorliegenden Erfindung in entriegeltem (unverschlossenen) Zustand und
Fig. 10 zeigt eine Perspektivenansicht des Verschluss-/Verriegelungsmechanismus gemäß drittem Ausführungsbeispiel in einem verriegelten (verschlossenen) Zustand.

### Figurenbeschreibung

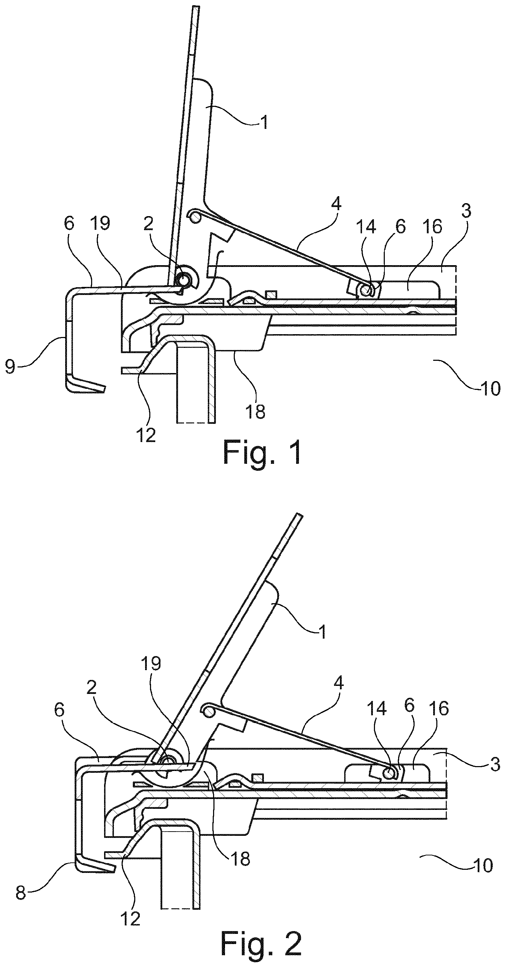

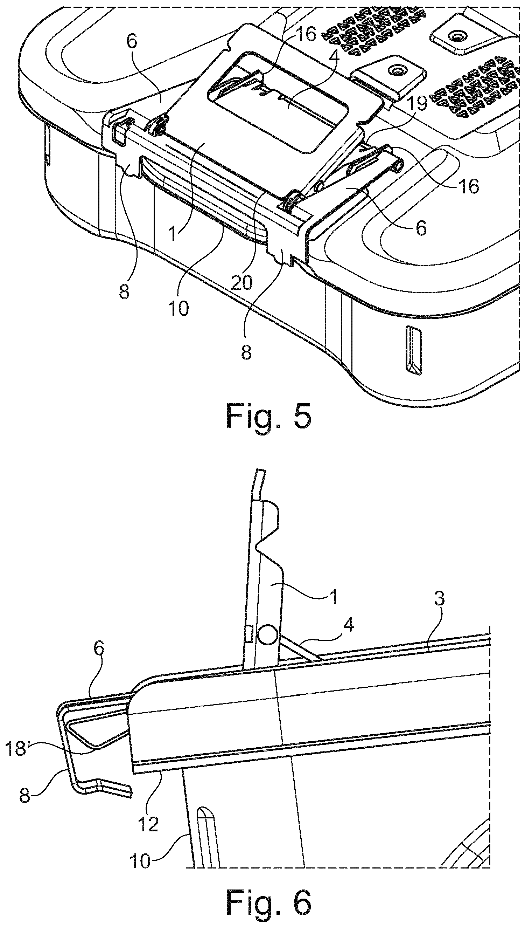

Ein Verriegelungs-/Verschlussmechanismus gemäß Fig. 1 bis 5 besteht im Wesentlichen aus einem vorzugsweise klappen- oder haltegriffförmigen Betätigungshebel 1, der an/im Bereich seiner einen Stirnkante an der Oberseite eines Containerdeckels 3 anscharniert ist. Vorzugsweise sind hierfür zwei axialbeabstandete Scharnierstifte 2 vorgesehen, die durch in Fig. 6 nur andeutungsweise gezeigte Ösen am Containerdeckel gesteckt und axial gesichert sind. In einem Mittenabschnitt des Betätigungshebels 1 sind zwei parallel beabstandete Stößel (Pleuelstangen) oder eine Stößelplatte 4 (siehe Fig. 6) endseitig angelenkt, die an ihrem andere Ende an einem Zugbügel/Kralle/Spannpratze/Verschlussschieber 6 anscharniert ist. Der Zugbügel 6 ist hierbei verschiebbar an der Oberseite des Containerdeckels 3 gelagert, derart, dass dieser in Abhängigkeit der Klappposition des Betätigungshebels 1 längsverschoben ist.

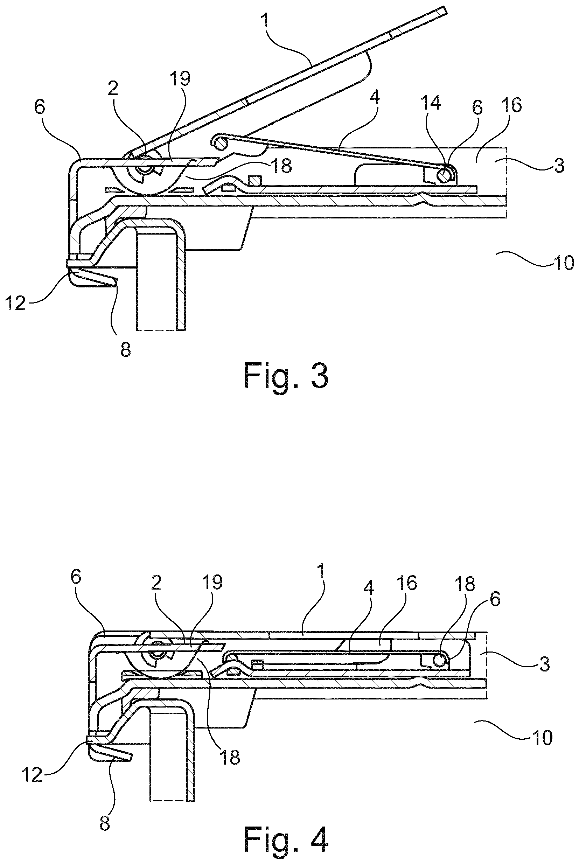

An seinem der Stößelanlenkstelle gegenüberliegenden, frei über die Umfangskante des Containerdeckels 3 auskragenden Ende hat/bildet der Zugbügel 6 eine in Richtung Deckelunterseite abgebogene Kralle/Haken 8, die dafür ausgebildet und vorgesehen ist, mit einer an einer Containerwanne 10 umfangsseitig ausgebildeten Leiste 12 in einen diese hinterschneidenden Wirkeingriff zu kommen, wie dies in den Fig. 3 und 4 besonders gut dargestellt ist.

Die Lagerung des Zugbügels 6 an der Oberseite des Containerdeckels 3 ist dergestalt, dass der Zugbügel 6 zum einen in seiner Längsrichtung verschiebbar und zum anderen an seinem frei vorkragenden Endabschnitt nach oben, d.h. weg von der Deckeloberseite bewegbar/verschwenkbar ist. Dies ist konstruktiv beispielsweise dadurch erreichbar, indem die Stößel /die Stößelplatte 4 jeweils an einer am Zugbügel 6 ausgeformten Öse (nicht näher gezeigt) anscharniert sind, indem durch die Ösen jeweils ein Scharnierzapfen oder eine durchgängige Scharnierstange 14 gesteckt ist, die wiederum in einer Führungskulisse 16 am Containerdeckel 3 gleitgeführt ist, welche ggf. auch einen fixen Endanschlag zumindest für die finale Entriegelungsposition der Betätigungshebels 1 definiert.

Unterhalb und/oder zwischen den Scharnierstiften 2 des Betätigungshebels 1 ist gemäß der Fig. 1 eine zu einem Bogen oder Trog gewölbte/gebogene Blattfeder 18 angeordnet, die sich demzufolge zwischen der Deckeloberseite und dem Zugbügel 6 bzw. dem Betätigungshebel 1 befindet. Dabei ist die Feder 18 so ausgerichtet, dass sie sich an ihrem konvex ausbauchenden Mittelabschnitt (direkt oder indirekt) gegen die Oberseite des Containerdeckels 3 abstützt/anlegt, wohingegen sich ihre freien Endkanten nach oben, d.h. in Richtung hin zum Betätigungshebel 1 erstrecken.

Der Zugbügel 6 hat ferner einen vorzugsweise zungenförmigen, in Richtung hin zu den Scharnierzapfen/Scharnierstange 14 sich erstreckenden Plattenabschnitt 19, der sich im Wesentlichen parallel zu der Oberseite des Containerdeckels 3 ausrichtet und dafür vorgesehen ist, die frei nach oben ragenden Endkanten der Feder 18 bei einer Betätigung des Betätigungshebels 1 in Verriegelungsrichtung zu übergleiten und dabei die Feder 18 zu plätten (biege-zu-spannen), um so eine Feder-Druckkraft in Richtung auf die dem Containerdeckel 3 zugewandte Unterseite des Zugbügels 6 (also nach oben) zu generieren.

Schließlich ist der Betätigungshebel 1 an seiner einen anscharnierten Stirnkante mit einer diese verlängernden bzw. über diese hinausragenden Niederdrücklasche 20 (siehe insbesondere Fig. 5 und Fig. 6) ausgebildet, die sich demzufolge zumindest in vollständig aufgeklapptem Zustand des Betätigungshebels 1 unterhalb der Scharnierstifte 2 (d.h. zwischen den Scharnierstiften 2 und dem Zugbügel 6) anordnet und so den Zugbügel 6 nach unten, d.h. in Richtung hin zur Oberseite des Containerdeckels 3 drückt.

Die Funktionsweise des Verschluss-/Verriegelungsmechanismus (Einheit) gemäß dem ersten bevorzugten Ausführungsbeispiel der vorliegenden Erfindung lässt sich wie folgt beschreiben:
In dem vollständig entriegelten/unverschlossenen Zustand des Verschluss-/Verriegelungsmechanismus gemäß Fig. 1 ist der Betätigungshebel 1 nahezu senkrecht zur Oberseite des Containerdeckels 3 ausgereichtet, wodurch der Spannbügel/Verschlussschieber 6 weit über die Umfangskante des Containerdeckels 3 hinausgeschoben ist, derart, dass dessen Haken oder Kralle 8 entfernt zur wannenseitigen Leiste 12 positioniert ist. Gleichzeitig ist der Plattenabschnitt 19 des Spannbügels/Verschlussschiebers 6 entfernt zu den frei nach oben ragenden Endkanten der Feder 18, sodass diese keine Federkraft auf den Spannbügel/Verschlussschieber 6 ausübt. Schließlich wird der Spannbügel/Verschlussschieber 6 durch die Niederdrücklasche 20 des Betätigungshebels 1 in Richtung hin zur Deckeloberseite gedrückt, derart, dass sich deren Kralle oder Haken 8 weit unterhalb der Leiste 12 befindet.

Sobald der Betätigungshebel 1 (manuell) umgelegt wird, bewirkt diese Schwenkbewegung über die Stößel/Stößelplatte 14 eine Translations-/Schiebebewegung des Spannbügels/Verschlussschiebers 6, wodurch sich die Kralle/Haken 8 des Spannbügels/Verschlussschiebers 6 parallel zur Deckeloberseite der wannenseitigen Leiste 12 annähert. Gleichzeitig nähert sich der Plattenabschnitt 19 des Spannbügels/Verschlussschiebers 6 den nach oben ragenden Endkanten der Feder 18 an.

Mit Erreichen einer im Wesentlichen halbverschlossenen/halbverriegelten Position gemäß der Fig. 3, 6 gleitet der Plattenabschnitt 19 schrittweise über die beiden Endkanten der Feder 18, um diese abzuflachen. Durch die dabei entstehende Federkraft wird der Spannbügel/Verschlussschieber 6 nach oben, d.h. weg von der Deckeloberseite gedrückt, wodurch sich der Haken/Kralle 8 des Spannbügels/Verschlussschiebers 6 an der Unterseite der wannenseitigen Leiste 12 anlegt und zunehmend eine Zugkraft auf diese ausübt. Hierdurch wird der Containerdeckel 3 zunehmend gegen die Oberkante der Containerwanne 10 gezogen.

In der Endposition gemäß der Fig. 4 hat der Betätigungshebel 1 geringfügig die Horizontale durch die Scharnierstifte 2 (Totpunktlinie) in Richtung hin zum Containerdeckel 3 überschritten und wird somit selbsthemmend in der Voll-Verriegelungsposition gehalten.

Dies bedeutet, dass die Betätigungskraft auf den Betätigungshebel 1 lediglich groß genug sein muss, den Spannbügel/Verschlussschieber 6 unter Überwindung von Reibkräften längs zu verschieben, wohingegen die eigentlichen Anziehkraft/Schließkraft zwischen Deckel 3 und Wanne 10 von der Feder 18 erzeugt wird. Dadurch, dass die Feder 18 trogartig um den/die Scharnierstift(e) 2 des Betätigungshebels 1 gebogen ist, benötigt diese nur wenig Bauraum und macht den gesamten Verschluss-/Verriegelungsmechanismus kompakt. Da ferner lediglich der Haken/Kralle 8 über den Umfangsrand des Deckels 3 in Richtung hin zur Wanne 10 vorkragt, wie die Wannenstirnseite nur geringfügig vom Verschluss-/Verriegelungsmechanismus, nämlich im Rahmen des Hakens/Kralle 8 auch bei einem groß dimensionierten Betätigungshebel 1 abgedeckt. Schließlich erlaubt der durch die Feder 18 erreichbare Federweg, der wesentlich größer ist als jener einer herkömmlichen, elastischen Dichtung zwischen Deckel und Wanne eine Art Überdruckventil-Funktion des Verschluss-/Verriegelungsmechanismus, indem bei Überschreiten Feder-abhängigen Werts des Container-Inndrucks der Deckel 3 entgegen der Federvorspannung durch die Feder 18 von der Wanne 10 abgehoben wird und so eine gewollte (großvolumige) temporäre Leckage entsteht.

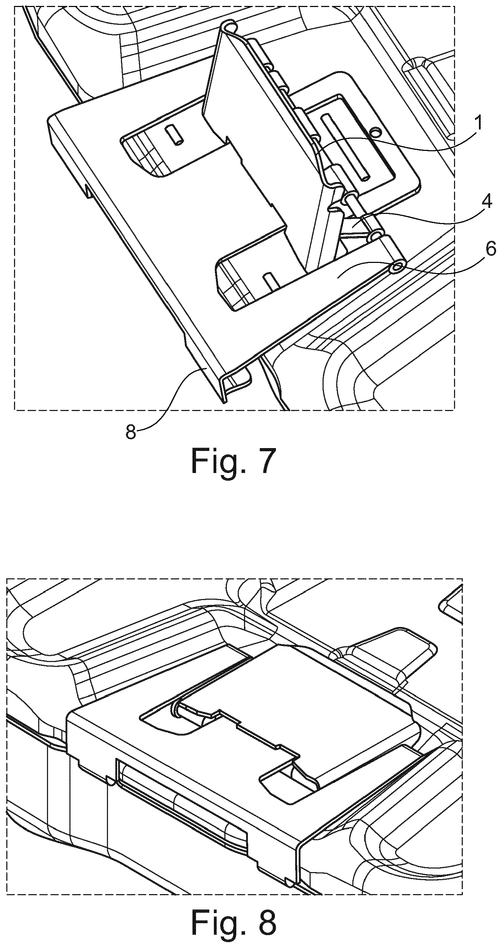

In Nachfolgenden wird ein zweites Ausführungsbeispiel der vorliegenden Erfindung anhand der Fig. 6 bis 8 beschrieben, wobei im Wesentlichen nur auf die zum ersten Ausführungsbeispiel unterschiedlichen konstruktiven Merkmale eingegangen wird. Bezüglich aller weiteren Merkmale und Funktionsweisen wird auf die vorstehende Figurenbeschreibung verwiesen.

Bei dem zweiten Ausführungsbeispiel ist die aus dem ersten Ausführungsbeispiel bekannte trogförmige Feder 18 durch eine am Spannbügel/Verschlussschieber 6 fixierte, sowie mit diesem sich bewegende/verschiebende Biegefeder 18'ersetzt. Im Konkreten ist gemäß der Fig. 6 an der der Deckeloberseite zugewandten Unterseite des Spannbügels/Verschlussschiebers 6 eine in der Seitenansicht keilförmig gebogene (Blatt-)Feder 18' fixiert, die in Richtung hin zu der Anlenkstelle des Stößels 4 an dem Spannbügel/Verschlussschieber 6 kontinuierlich und/oder stufenartig spitz zuläuft. Die Umfangskante des Containerdeckels 3 bildet hierbei eine Abgleitbahn für die Feder 18'.

Wird demzufolge der Spannbügel/Verschlussschieber 6 für ein Verriegeln des Containers über den Betätigungshebel 1 verschoben, gleitet die Feder 18' über die Abgleitbahn ausgehend von der Federspitze in Richtung hin zu Ihrem keilformentsprechenden maximalen Höhenabschnitt hinweg, wodurch sich die auf den Spannbügel/Verschlussschieber 6 ausgeübte Feder-Druckkraft erhöht und somit der Haken/Kralle 12 an der Unterseite der Leiste 12 andrückt. Dadurch wird der Deckel 3 gegen die Wanne 10 gezogen, wie dies anhand des ersten Ausführungsbeispiels bereits beschrieben ist.

Durch das zweite Ausführungsbeispiel werden funktional die gleichen Vorteile erzielt wir beim ersten Ausführungsbeispiel, wobei jedoch im Gegensatz hierzu die Feder 18' zumindest in der unverriegelten Stellung des Verriegelungs-/Verschlussmechanismus exponiert ist.

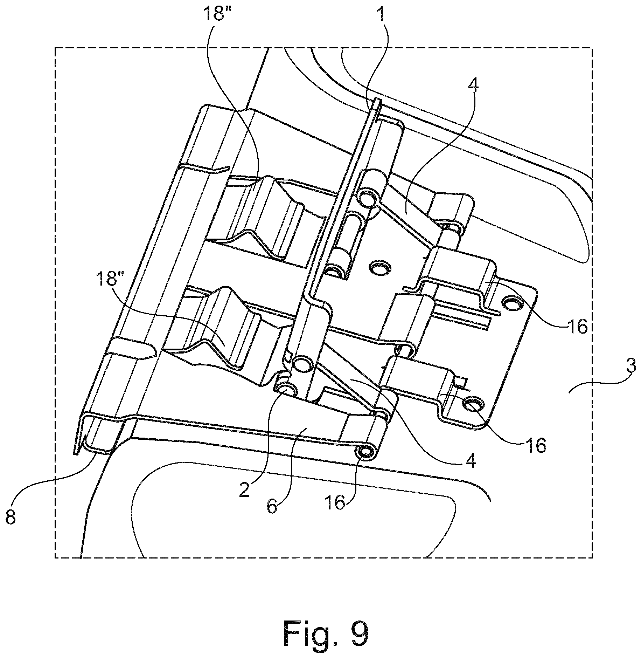

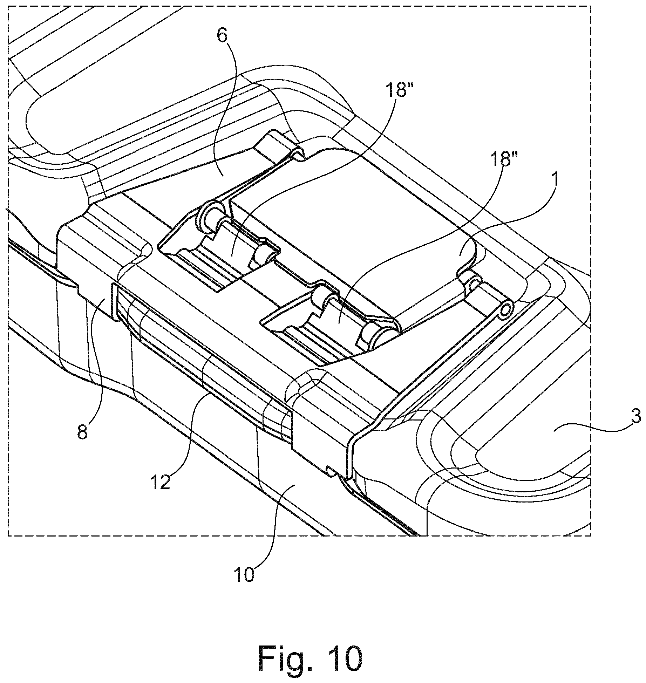

In Nachfolgenden wird ein drittes Ausführungsbeispiel der vorliegenden Erfindung anhand der Fig. 9 und 10 beschrieben, wobei im Wesentlichen nur auf die zum ersten und zweiten Ausführungsbeispiel unterschiedlichen konstruktiven Merkmale eingegangen wird. Bezüglich aller weiteren Merkmale und Funktionsweisen wird auf die vorstehende Figurenbeschreibung verwiesen.

Wie vorstehend ausgeführt wurde, ist dort die Feder 18' am Spannbügel/Verschlussschieber 6 fixiert, um sich gemeinsam mit diesem zu verschieben. Demzufolge ist es alternativ hierzu aber auch möglich, eine entsprechende Feder 18" am Containerdeckel 3 zu fixieren, wie dies insbesondere in der Fig. 9 gezeigt ist.

In anderen Worten ausgedrückt, sieht das dritte Ausführungsbeispiel der vorliegenden Erfindung vor, den Spannbügel/Verschlussschieber 6 mit zumindest einem, vorzugsweise zwei fensterartigen Ausbrüchen auszubilden, in deren jeweiligem Bereich eine gewellte oder einfach gekrümmte Blattfeder 18" am Containerdeckel 3 montiert ist, derart, dass jede Feder 18" mit ihrem jeweiligen konvex gewölbten Mittenabschnitt durch den zugehörigen fensterartigen Ausbruch hindurch nach oben (von der Deckeloberseite weg) vorragt, wenn sich der Verschluss-/Verriegelungsmechanismus ein einer entriegelten/unverschlossenen Position gemäß der Fig. 9 befindet. In diesem Zustand wird der Spannbügel/Verschlussschieber 6 durch die Niederdrücklasche 20 am Betätigungshebel 1 nach unten in Richtung hin zur Deckeloberseite gedrückt, ohne dass eine gegensätzliche Federkraft auf diesen erzeugt wird.

Sobald aber der Betätigungshebel 1 in Richtung Verriegelungsposition verschwenkt und dadurch der Spannbügel/Verschlussschieber 6 verschoben wird, wandern die gebogenen Blattfedern 18" aus den jeweiligen fensterartigen Ausbrüchen heraus, d.h. der Spannbügel/Verschlussschieber 6 gleitet zunehmend über die die beiden Blattfedern 18" wodurch diese abgeplättet werden und demzufolge eine Druckkraft auf die Unterseite des Spannbügels/Verschlussschiebers 6 aufbringen. Auch in diesem Fall können sämtliche Vorteile des ersten Ausführungsbeispiels verwirklicht werden.

## Patentansprüche

1. Verschluss-/ Verriegelungsmechanismus mit:
einem vorzugsweise schwenkbar gelagerten Betätigungshebel (1);
einem Zugbügel oder Kralle/Spannpratze (6), die mit dem Betätigungshebel (1) direkt oder indirekt gekoppelt ist; und
einem eine Hinterschneidung ausbildenden Eingriffselement (12), mit welchem der Zugbügel oder Kralle/Spannpratze (6) in Wirkeingriff bringbar ist, um bei einer Betätigung des Betätigungshebels (1) eine Zugkraft auf das Eingriffselement (12) auszuüben;
**dadurch gekennzeichnet, dass**
der Zugbügel oder Kralle/Spannpratze (6) quer, vorzugsweise in einem im Wesentlichen rechten Winkel, zur auszuübenden Zugkraft verschiebbar gelagert ist; und
eine Feder (18, 18', 18") oder dergleichen Vorspannelement relativ zu dem Zugbügel oder Kralle/Spannpratze (6) derart in einer Wirkposition angeordnet ist, dass diese erst bei einer vorbestimmten Betätigungsstellung des Betätigungshebels (1) und demzufolge einer vorbestimmten bzw. vorbestimmbaren Verschiebeposition des Zugbügels oder Kralle/Spannpratze (6), in welcher der Zugbügel oder Kralle/Spannpratze (6) das Eingriffselement (12) bereits hintergreift, eine Druckkraft auf den Zugbügel oder Kralle/Spannpratze (6) in Richtung im Wesentlichen parallel zu der auszuübenden Zugkraft anlegt.

2. Verschluss-/Verriegelungsmechanismus nach Anspruch 1, **dadurch gekennzeichnet, dass** der Betätigungshebel (1) über zumindest einen daran angelenkten Stößel oder Stößelplatte (4) mit dem Zugbügel oder Kralle/Spannpratze (6) für eine Transformation der Schwenkbewegung des Betätigungshebels (1) in eine Translationsbewegung des Zugbügels oder Kralle/Spannpratze (6) gekoppelt ist.

3. Verschluss-/Verriegelungsmechanismus nach Anspruch 2, **dadurch gekennzeichnet, dass** der Betätigungshebel (1) über zumindest einen, vorzugsweise zwei axial beabstandete Scharnierstifte (2) an einem Containerdeckel (3) angelenkt ist, derart, dass sich der Zugbügel oder die Kralle/Spannpratze (6) bezüglich des Betätigungshebels (1) unterhalb des zumindest einen, vorzugsweise zwischen den zwei axial beabstandeten Scharnierstiften (2) erstreckt.

4. Verschluss-/Verriegelungsmechanismus nach Anspruch 3, **dadurch gekennzeichnet, dass** der Betätigungshebel (1) an seinem einen freien Endabschnitt eine Betätigungsplatte oder Betätigungsgriff und an seinem anderen freien Endabschnitt eine Niederdrücklasche (20) bildet, welche bei aufgeklapptem Betätigungshebel (1) den Zugbügel oder Kralle/Spannpratze (6) bezüglich des/der Scharnierstift(e) (2) niederdrückt und bei eingeklapptem Betätigungshebel den Zugbügel oder Kralle/Spannpratze für dessen Bewegung hin zu dem/den Scharnierstift(en) (2) freigibt.

5. Verschluss-/Verriegelungsmechanismus nach Anspruch 3, **dadurch gekennzeichnet, dass** um den/die Scharnierstift(e) (2) bzw. deren Längsachse herum die Feder (18) als Blattfeder sichel- oder teilkreisförmig gebogen ist, derart, dass sich diese an ihrer einen freien Blattfederkante an einer bezüglich des/der Scharnierstifts (2) unteren Seite des Zugbügels (6) oder Kralle/Spannpratze anliegt und an ihrer anderen freien Blattfederkante gegen eine in Schieberichtung des Zugbügels oder Kralle/Spannpratze sich erstreckende Zunge (19) des Zugbügels oder Kralle/Spannpratze (6) erst dann andrückt, wenn dieser die vorbestimmte Verschiebeposition erreicht, um so an ihrer einen freien Blattfederkante die Druckkraft auf den Zugbügel oder Kralle/Spannpratze (6) anzulegen, wohingegen der Zugbügel oder die Kralle/Spannpratze (6) in einer Position außerhalb oder vor der vorbestimmten/vorbestimmbaren Verschiebeposition vollständig vorspannfrei ist.

6. Verschluss-/Verriegelungsmechanismus nach Anspruch 1, **dadurch gekennzeichnet, dass** die Feder (18') vorzugsweise in Form einer Bügelfeder am Zugbügel oder Kralle/Spannpratze (6) fixiert ist und sich mit diesem mitverschiebt, derart, dass sich diese bei einer Verschiebung des Zugbügels oder Kralle/Spannpratze (6) an einem Containerdeckel (3) erst dann federelastisch abstützt, um so die Druckkraft auf den Zugbügel oder Kralle/Spannpratze (6) anzulegen, wenn der Zugbügel oder Kralle/Spannpratze (6) die vorbestimmte Verschiebeposition erreicht oder erreicht hat, wohingegen der Zugbügel oder die Kralle/Spannpratze (6) in einer Position außerhalb oder vor der vorbestimmten/vorbestimmbaren Verschiebeposition vollständig vorspannfrei ist.

7. Verschluss-/Verriegelungsmechanismus nach Anspruch 1, **dadurch gekennzeichnet, dass** die Feder (18"), vorzugsweise in Form einer gewellten oder einfach gewölbten Blattfeder, an einem Containerdeckel (3) fixiert ist, derart, dass diese vom Zugbügel oder Kralle/Spannpratze (6) überglitten wird, zumindest wenn dieser die vorbestimmte Verschiebeposition erreicht, um so die Druckkraft auf den Zugbügel oder Kralle/Spannpratze (6) anzulegen, wohingegen der Zugbügel oder die Kralle/Spannpratze (6) in einer Position außerhalb oder vor der vorbestimmten/vorbestimmbaren Verschiebeposition vollständig vorspannfrei ist.

8. Verschluss-/Verriegelungsmechanismus nach Anspruch 1, **dadurch gekennzeichnet, dass** der Zugbügel oder Kralle/Spannpratze (6) in seiner oder ihrer Längsrichtung längsverschiebbar an einem Containerdeckel (3) oder einer Containerwanne (10) gelagert ist.

9. Verschluss-/Verriegelungsmechanismus nach Anspruch 1, **dadurch gekennzeichnet, dass** die auf das Eingriffselement (12) für ein Zusammenpressen einer Containerwanne (10) und eines Containerdeckels (3) auszuübende Zugkraft ausschließlich durch die Feder (18, 18', 18") oder dergleichen Vorspannelement erzeugt wird.

10. Medizinischer Sterilcontainer mit einem Verschluss-/Verriegelungsmechanismus gemäß einem der Patentansprüche 1 bis 9.

## Claims

1. A closing/locking mechanism, comprising:
an actuation lever (1), preferably pivotably mounted;
a tension bow or claw/clamping claw (6), which is directly or indirectly coupled to the actuation lever (1); and
an engagement element (12) forming an undercut, with which the tension bow or claw/clamping claw (6) can be brought into operative engagement in order to exert a tensile force on the engagement element (12) when the actuation lever (1) is actuated;
**characterized in that**
the tension bow or claw/clamping claw (6) is mounted so as to be displaceable transversely, preferably at a substantially right angle, to the tensile force to be exerted; and
a spring (18, 18', 18") or a similar pre-tensioning element is arranged in an operative position relative to the tensioning bow or claw/clamping claw (6) in such a way that it only applies a compressive force to the tension bow or claw/clamping claw (6) in a direction essentially parallel to the tensile force to be exerted, when the actuation lever (1) is in a predetermined actuating position and consequently in a predetermined or predeterminable displacement position of the tension bow or claw/clamping claw (6), in which the tension bow or claw/clamping claw (6) already egages behind the engagement element (12).

2. The closing/locking mechanism according to claim 1, **characterized in that** the actuation lever (1) is coupled to the tension bow or claw/clamping claw (6) via at least one tappet or tappet plate (4) hinged thereto for transformation of the pivot motion of the actuation lever (1) to a translational motion of the tension bow or claw/clamping claw (6).

3. The closing/locking mechanism according to claim 2, **characterized in that** the actuation lever (1) is hinged to a container lid (3) via at least one, preferably two axially spaced hinge pins (2), in such a way that the tension bow or the claw/clamping claw (6) extends below the at least one, preferably between the two axially spaced hinge pins (2) with respect to the actuation lever (1).

4. The closing/locking mechanism according to claim 3, **characterized in that** the actuation lever (1) forms an actuation plate or actuation handle at its one free end portion, and a push-down lug (20) at its other free end portion, which, when the actuation lever (1) is folded open, presses down the tension bow or claw/clamping claw (6) with respect to the hinge pin(s) (2) and, when the actuation lever is folded in, releases the tension bow or claw/clamping claw for its motion towards the hinge pin(s) (2).

5. The closing/locking mechanism according to claim 3, **characterized in that** around the hinge pin(s) (2) or their longitudinal axis, the spring (18) is bent as a leaf spring in a sickle-shaped or semi-circular manner such that at its one free leaf spring edge, it is in contact with a lower side of the tension bow (6) or claw/clamping claw with respect to the hinge pin(s) and at its other free leaf spring edge presses against a tongue (19) of the tension bow or claw/clamping claw (6) extending in a pushing direction of the tension bow or claw/clamping claw when the latter has reached the predetermined displacement position in order to apply the compressive force to the tension bow or claw/clamping claw (6) at its one free leaf spring edge, whereas the tension bow or claw/clamping claw (6) is completely free of pretension in a position outside or before the predetermined/predeterminable displacement position.

6. The closing/locking mechanism according to claim 1, **characterized in that** the spring (18') is preferably fixed to the tension bow or claw/clamping claw (6) in the form of a yoke spring and moves with it in such a way that, when the tension bow or claw/clamping claw (6) is displaced, it is only then resiliently supported on a container lid (3) so as to thereby apply a compressive force to the tension bow or claw/clamping claw (6) when the tension bow or claw/clamping claw (6) reaches or has reached the predetermined displacement position, whereas the tension bow or claw/clamping claw (6) is completely free of pretension in a position outside or before the predetermined/predeterminable displacement position.

7. The closing/locking mechanism according to claim 1, **characterized in that** the spring (18"), preferably in the form of a corrugated or simply curved leaf spring, is fixed to a container lid (3) such that it is slid over by the tension bow or claw/clamping claw (6) at least when the latter reaches the predetermined displacement position to thereby apply a compressive force to the tension bow or claw/clamping claw, whereas the tension bow or claw/clamping claw (6) is completely free of pretension in a position outside or before the predetermined/predeterminable displacement position.

8. The closing/locking mechanism according to claim 1, **characterized in that** the tension bow or claw/clamping claw (6) is mounted on a container lid (3) or a container trough (10), so as to be longitudinally displaceable in its longitudinal direction.

9. The closing/locking mechanism according to claim 1, **characterized in that** the tensile force to be exerted on the engagement element (12) for pressing together a container trough (10) and a container lid (3) is generated exclusively by the spring (18, 18', 18") or a similar pretensioning element.

10. A medical sterile container including a closing/locking mechanism according to any of claims 1 to 9.

## Revendications

1. Mécanisme de fermeture/verrouillage avec :
un levier d'actionnement (1) logé de préférence de manière pivotante ;
un étrier de traction ou une griffe/griffe de serrage (6) qui est couplé(e) directement ou indirectement au levier d'actionnement (1) ; et
un élément de prise (12) formant une contre-dépouille, avec lequel l'étrier de traction ou la griffe/griffe de serrage (6) peut être amené(e) en prise active afin d'exercer lors d'un actionnement du levier d'actionnement (1) une force de traction sur l'élément de prise (12) ;
**caractérisé en ce que**
l'étrier de traction ou la griffe/griffe de serrage (6) est logé(e) de manière mobile transversalement, de préférence dans un angle sensiblement droit, à la force de traction à exercer ; et
un ressort (18, 18', 18") ou élément de précontrainte similaire par rapport à l'étrier de traction ou la griffe/griffe de serrage (6) est agencé dans une position active de telle manière que celui-ci applique seulement pour une position d'actionnement prédéterminée du levier d'actionnement (1) et par conséquent une position de déplacement prédéterminée ou pouvant être prédéterminée de l'étrier de traction ou la griffe/griffe de serrage (6), dans laquelle l'étrier de traction ou la griffe/griffe de serrage (6) vient déjà en prise derrière l'élément de prise (12), une force de pression sur l'étrier de traction ou la griffe/griffe de serrage (6) en direction sensiblement parallèle à la force de traction à exercer.

2. Mécanisme de fermeture/verrouillage selon la revendication 1, **caractérisé en ce que** le levier d'actionnement (1) est couplé par le biais d'au moins un coulisseau ou une plaque de coulisseau (4) articulée à celui-ci avec l'étrier de traction ou la griffe/griffe de serrage (6) pour une transformation du mouvement de pivotement (1) en un déplacement de translation de l'étrier de traction ou la griffe/griffe de serrage (6).

3. Mécanisme de fermeture/verrouillage selon la revendication 2, **caractérisé en ce que** le levier d'actionnement (1) est articulé par le biais d'au moins une, de préférence deux tiges de charnière (2) espacées axialement à un couvercle de contenant (3) de telle manière que l'étrier de traction ou la griffe/griffe de serrage (6) s'étende par rapport au levier d'actionnement (1) en dessous de l'au moins une, de préférence entre les deux tiges de charnière (2) espacées axialement.

4. Mécanisme de fermeture/verrouillage selon la revendication 3, **caractérisé en ce que** le levier d'actionnement (1) forme au niveau d'une de ses sections d'extrémité libres une plaque d'actionnement ou prise d'actionnement et au niveau de son autre section d'extrémité libre une languette d'enfoncement (20) qui enfonce en cas de levier d'actionnement (1) déplié l'étrier de traction ou la griffe/griffe de serrage (6) par rapport à la/aux tige(s) de charnière (2) et libère en cas de levier d'actionnement replié l'étrier de traction ou la griffe/griffe de serrage pour son déplacement vers la/les tige(s) de charnière (2).

5. Mécanisme de fermeture/verrouillage selon la revendication 3, **caractérisé en ce qu'** autour de la/des tige(s) de charnière (2) ou son axe longitudinal, le ressort (18) est plié comme ressort à lames en forme de croissant ou de cercle partiel de telle manière que celui-ci repose contre une de ses arêtes de ressort à lames libre au niveau d'un côté inférieur par rapport à la/aux tige(s) de charnière (2) de l'étrier de traction (6) ou la griffe/griffe de serrage et presse contre son autre arête de ressort à lames libre contre une languette (19) s'étendant dans le sens de coulissement de l'étrier de traction ou la griffe/griffe de serrage de l'étrier de traction ou la griffe/griffe de serrage (6) seulement lorsque celui-ci atteint la position de déplacement prédéterminée afin d'appliquer ainsi sur une de ses arêtes de ressort à lames libres la force de pression sur l'étrier de traction ou la griffe/griffe de serrage (6), alors que l'étrier de traction ou la griffe/griffe de serrage (6) est complètement exempt(e) de précontrainte dans une position en dehors ou avant la position de déplacement prédéterminée/pouvant être prédéterminée.

6. Mécanisme de fermeture/verrouillage selon la revendication 1, **caractérisé en ce que** le ressort (18') est de préférence fixé sous la forme d'un ressort en étrier à l'étrier de traction ou la griffe/griffe de serrage (6) et se déplace avec celui-ci de telle manière que celui-ci s'appuie lors d'un déplacement de l'étrier de traction ou la griffe/griffe de serrage (6) contre un couvercle de contenant (3) seulement de manière élastique afin d'appliquer ainsi la force de pression sur l'étrier de traction ou la griffe/griffe de serrage (6) lorsque l'étrier de traction ou la griffe/griffe de serrage (6) atteint ou a atteint la position de déplacement prédéterminée, alors que l'étrier de traction ou la griffe/griffe de serrage (6) est complètement exempt(e) de précontrainte dans une position en dehors ou avant la position de déplacement prédéterminée/pouvant être prédéterminée.

7. Mécanisme de fermeture/verrouillage selon la revendication 1, **caractérisé en ce que** le ressort (18") est de préférence fixé sous la forme d'un ressort à lames ondulé ou simplement courbé au couvercle de contenant (3) de telle manière que l'étrier de traction ou la griffe/griffe de serrage (6) glisse sur celui-ci, au moins lorsque celui-ci atteint la position de déplacement prédéterminée afin d'appliquer ainsi la force de pression sur l'étrier de traction ou la griffe/griffe de serrage (6), alors que l'étrier de traction ou la griffe/griffe de serrage (6) est complètement exempt(e) de précontrainte dans une position en dehors ou avant la position de déplacement prédéterminée/pouvant être prédéterminée.

8. Mécanisme de fermeture/verrouillage selon la revendication 1, **caractérisé en ce que** l'étrier de traction ou la griffe/griffe de serrage (6) est logé(e) dans son sens longitudinal de manière mobile longitudinalement au niveau d'un couvercle de contenant (3) ou une cuve de contenant (10).

9. Mécanisme de fermeture/verrouillage selon la revendication 1, **caractérisé en ce que** la force de traction à exercer sur l'élément de prise (12) pour une compression d'une cuve de contenant (10) et d'un couvercle de contenant (3) est générée exclusivement par le ressort (18, 18', 18") ou l'élément de précontrainte similaire.

10. Contenant stérile médical avec un mécanisme de fermeture/verrouillage selon l'une quelconque des revendications 1 à 9.
